# EUROPEAN PATENT APPLICATION

(11) **EP 1 925 213 A1**
(43) Date of publication of application: **28.05.2008**
(21) Application number: 06024383.9
(22) Date of filing: 24.11.2006
(51) Int. Cl.: A23L 1/30, A61K 36/537, A61P 25/24, A61P 25/28

(54) **Dietary and pharmaceutical compositions comprising a sage extract comprising a mixture of tricyclic diterpenes and their derivatives and their uses**

(71) Applicant: DSM IP Assets B.V., 6411 TE Heerlen (NL)
(72) Inventor: The designation of the inventor has not yet been filed
(74) Representative: Steck, Melanie

(57) **Abstract**

The present invention refers to a sage extract containing a mixture of tricyclic diterpene (derivative)s of the formulae I to III, preferably to a sage extract containing a mixture of tricyclic diterpene (derivative)s of the formulae I to IV, more preferably to a sage extract containing a mixture of tricyclic diterpene (derivative)s of the formulae I to V, for use as medicaments for the treatment of a disorder connected to impaired neurotransmission, as well as to dietary and pharmaceutical compositions and their uses.

## Description

The present invention refers to a sage extract comprising a mixture of tricyclic diterpenes and their derivatives for use as medicaments, especially for the treatment of disorders connected to impaired neurotransmission, as well as to dietary and pharmaceutical compositions containing such sage extract and their uses.

It is well known that impaired neurotransmission, e.g. low neurotransmitter levels, is connected to mental diseases such as depression and generalized anxiety disorders (GAD), and increased susceptibility to stress.

Compounds that increase neurotransmitter levels in the brain and thus enhance their transmission, exhibit therefore antidepressant properties as well as beneficial effects on a variety of other mental disorders ("Neurotransmitters, drugs and brain function" R.A. Webster (ed), John Wiley & Sons, New York, 2001, p. 187-211, 289-452, 477-498). The main neurotransmitters are serotonin, dopamine, noradrenaline (= norepinephrine), acetylcholine, glutamate, gamma-amino-butyric acid, as well as neuropeptides. Increase in neurotransmission is achieved by increasing the concentration of the neurotransmitter in the synaptic cleft thus making it available for increased or prolonged neurotransmission through inhibition of re-uptake into the pre-synaptic nerve end, or by preventing neurotransmitter catabolism by inhibition of degrading enzymes such as monoaminooxidase A and B.

The term "impaired neurotransmission" is used in the present application in accordance with its meaning well-known to the person skilled in the art and relates to a deregulation of neurotransmission and which may occur at the level of neurotransmitter biosynthesis, processing, storage, release re-uptake and receptor binding. Impaired neurotransmission may manifest itself in animals including humans as a disturbance of behaviour, emotions, mood and thinking processes, for example, in one of various types of depression.

Tricyclic antidepressant compounds (TCAs) such as imipramine, amitriptyline, and clomipramine e.g. inhibit the re-uptake of serotonin and noradrenaline. They are widely regarded as among the most effective antidepressants available, but they have a number of disadvantages because they interact with a number of brain receptors, e.g. with cholinergic receptors. Most importantly, TCAs are not safe when taken in overdose, frequently showing acute cardiotoxicity.

Another class of antidepressant drugs are the so-called SSRIs (selective serotonin re-uptake inhibitors) including fluoxetine, paroxetine, sertraline, citalopram, fluvoxamine that block the serotonin transporter (SERT), a high affinity sodium chloride-dependent neurotransmitter transporter that terminates serotonergic neurotransmission by uptake of serotonin. They have been proven as effective in the treatment of depression and anxiety, and are usually better tolerated than TCAs. These medications are typically started at low dosages and may be increased until they reach a therapeutic level. A common side effect is nausea. Other possible side effects include decreased appetite, dry mouth, sweating, infection, constipation, yawn, tremor, sleepiness and sexual dysfunction.

In addition, compounds that prevent the catabolism of neurotransmitters more broadly by inhibiting the monoaminooxidases (MAOs) A and B exhibit antidepressant effects. MAOs catalyse the oxidation of amino group containing neurotransmitters such as serotonin, noradrenaline, and dopamine.

Furthermore, modulators of neurotransmission exert pleiotropic effects on mental and cognitive functions.

There is a need for compounds for the treatment or prevention of mental diseases and/or disorders which do not show the negative side effects of known antidepressants. Many patients are interested in alternative therapies which could minimize the side effects associated with high-dose of drugs and yield additive clinical benefits. Severe depression is a long lasting and recurring disease, which is usually poorly diagnosed. Furthermore many patients suffer from mild or middle severe depression. Thus, there is an increasing interest in the development of compounds as well as pharmaceutical and/or dietary compositions that may be used to treat mental diseases/disorders or to prevent the development of mental diseases/disorders such as depression and dysthymia in people at risk, to stabilize mood and achieve emotional balance.

In addition, patients often suffer either as a comorbidity to depression, or by itself from generalized anxiety syndrome (GAD). GAD is a highly prevalent anxiety condition and chronic illness in primary care (~10% of patients) (Wittchen, HU and Jacoby, F. European Neuropsychopharmacology 15 (2005) 357 - 376). Patients present themselves to their primary care physician with multiple physical symptoms. GAD is characterized by chronic tension, and anxious worrying and tension (> 6 months), which are disabling and uncontrollable, and accompanied by a characteristic hypervigilance syndrome (including restlessness, muscle tension, and sleep problems). If untreated, GAD runs a chronic, fluctuating course and tends to get more severe with age. GAD patients suffer from subsyndromal depression and contribute to the highest overall direct and indirect health economic burden of all anxiety and depressive disorder. Despite high GAD incidence, few sufferers are diagnosed, prescribed medication, or receive psychiatric referral-simple diagnostic tools to aid patient recognition and monitoring are needed. Regardless of specific diagnosis, physicians require effective GAD-symptom treatments. SSRIs suche as paroxetine are effective for GAD treatment [Stocchi et al., Journal of Clinical Psychiatry, Efficacy and tolerability of paroxetine for the long-term treatment of generalized anxiety disorder, 2003, 63(3), 250-258]. Also, systematic reviews and placebo-controlled RCTs (Randomized Clinical Trials) indicate that some SSRIs (escitalopram, paroxetine and sertraline), the SNRI (Selective Norepinephrine Reuptake Inhibitors) venlafaxine, some benzodiazepines (alprazolam and diazepam), the tricyclic imipramine, and the 5-HTI A partial agonist buspirone are all efficacious in acute treatment. In general, the effect of treatment is often moderate and symptoms reappear when the treatment period is discontinued. Therefore, a continuous long-term treatment or prevention with compounds which have less side effects as SSRIs and can be taken over long time periods might by favourable over drug treatment.

Mood disorders and occupational stress also lead to consecutive sleep disorders, insomnia, low sleep quality, disturbances in circadian rhythms. These conditions are often chronic and can persist over long time. Also, deregulation of circadian rhythms induced by long-term flights (jet-lag) as well as by shift-working can cause similar symptoms and distress. Therefore, treatment with dietary supplementation to alleviate and prevent symptoms associated with the sleep disorders, such as impairment of cognitive function and memory, mental and physical fatigue, dreaminess, is warranted to improve the overall quality of life and benefiting vital energy of a person in need thereof.

According to the present invention this demand is met with a sage extract comprising the following tricyclic diterpene (derivative)s of the formulae I to III, i.e. carnosol (compound of formula I), carnosic acid (compound of formula II) and camosic acid 12-methyl ether (compound of formula III).

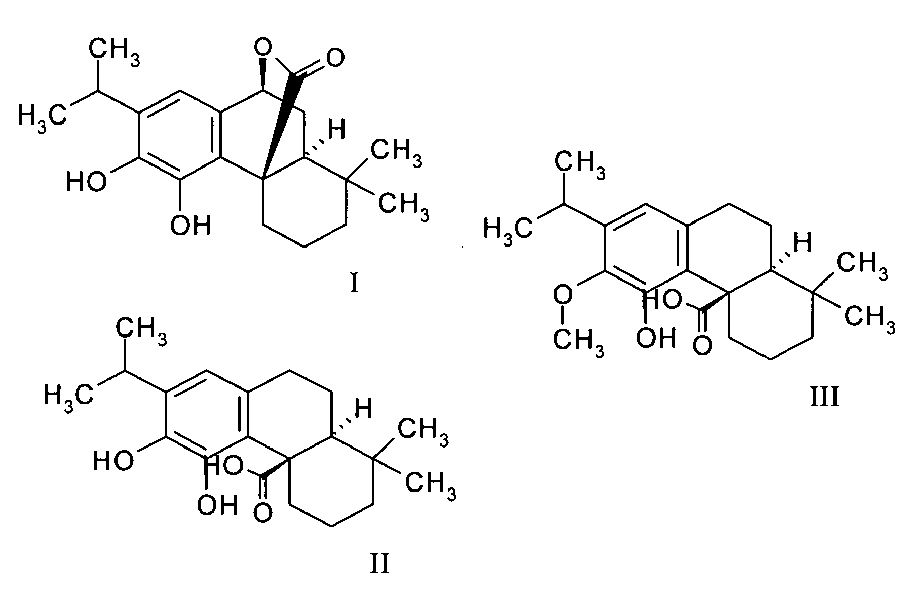

Optionally rosmanol (compound of the formula IV) and/or 20-deoxocarnosol (compound of the formula V) may also be present.

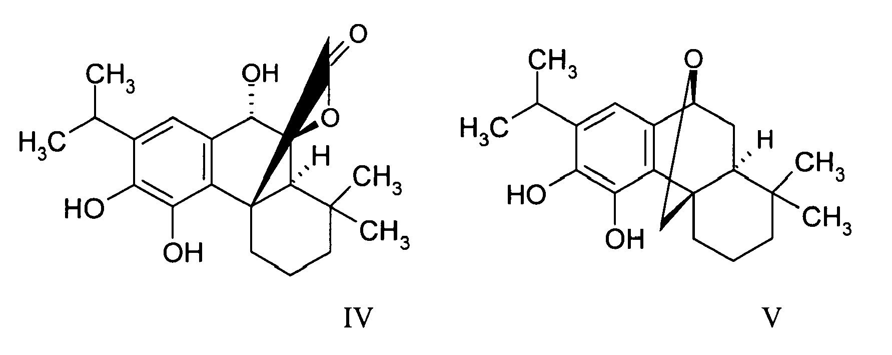

Especially preferred are those sage extracts that (beside carnosol, carnosic acid and carnosic acid 12-methyl ether) additionally contain also rosmanol.

The amounts of these tricyclic diterpenes in the sage extract may vary in the range of from 0.1 to 15 weight-% (preferably from 1 to 12 weight-%, more preferably from 9 to 11 weight-%) for camosic acid, in the range of from 0.01 to 10 weight-% (preferably from 2 to 8 weight-%, more preferably from 2 to 5 weight-%) for camosol and in the range of from 0.1 to 7 weight-% (preferably from 0.2 to 6 weight-%, more preferably from 3 to 5 weight-%) for camosic acid 12 methyl ether, based on the total weight of the sage extract, whereby camosic acid is preferably the main component. If rosmanol is present its amount may vary in the range of from 0.1 to 1.0 weight-%, preferably from 0.1 to 0.7 weight-%, more preferably from 0.1 to 0.5 weight-%. If 20-deoxo-carnosol is present its amount may be below 1.0 weight-%, preferably it may vary in the range of from 0.01 to 1.0 weight-%, more preferably from 0.05 to 0.8 weight-%, most preferably from 0.07 to 0.5 weight-%.

Thus, in one aspect the invention relates to a sage extract containing such tricyclic diterpene (derivative)s I, II and III, preferably to a sage extract containing such a mixture of the tricyclic diterpene (derivative)s I, II, III and IV, more preferably to a sage extract containing such a mixture of the tricyclic diterpene (derivative)s I to V, for use as medicament for the treatment of a disorder connected to impaired neurotransmission.

In another aspect, the invention relates to the use of a sage extract containing the mixture of the tricyclic diterpene (derivative)s with the definitions and preferences as described above for the manufacture of a composition for the treatment of a disorder connected to impaired neurotransmission, particularly for the manufacture of an antidepressant, a mood/vitality improver, a stress reliever, a condition improver, a reducer of anxiety, a reducer of obsessive-compulsive behaviour, a relaxant, a sleep improver and/or a insomnia alleviator.

In still another aspect, the invention relates to a dietary composition containing a sage extract containing the mixture of the tricyclic diterpene (derivative)s with the definitions and preferences as described above as well as to a pharmaceutical composition containing a sage extract containing the mixture of the tricyclic diterpene (derivative)s with the definitions and preferences as described above and a conventional pharmaceutical carrier.

Further, the invention relates to a method for the treatment of a disorder connected to impaired neurotransmission in animals including humans, said method comprising administering an effective dose of a sage extract containing the mixture of the tricyclic diterpene (derivative)s with the definitions and preferences as described above to animals including humans which are in need thereof.

Animals in the context of the present invention include humans and encompass mammals, fish and birds. Preferred "animals" are humans, pet animals and farm animals.

Examples of pet animals are dogs, cats, birds, toy fish, guinea pigs, (jack) rabbits, hares and ferrets. Examples of farm animals are fish, pigs, horses, ruminants (cattle, sheep and goat) and poultry.

The term "sage extract" means any extract of sage containing the tricyclic diterpene (derivative)s I, II and III, preferably containing the tricyclic diterpene (derivative)s I, II, III and IV, more preferably containing the tricyclic diterpene (derivative)s I, II, III, IV and V. Suitable sage species are Salvia officinalis, Salvia miltiorhiza, Salvia lanigera, Salvia canariensis, Salvia fruticosa, Salvia mellifera, Salvia tomentosa, Salvia deserta, Salvia przewalskii, and Salvia sclarea. Preferred is Salvia officinalis.

Sage extracts suitable for the uses of the present invention are e.g. commercially available from FLAVEX Naturextrakte GmbH, Rehlingen, Germany, as the "Salbei Extrakt, Typ Nr. 063.001" containing 0.06 weight-% of carnosol, 0.14 weight-% of carnosic acid and 0.36 weight-% of carnosic acid 12-methyl ether, based on the total weight of the sage extract (measured by HPLC-UV at 210 nm with the pure substances as reference); and the "Salbei Antioxidans Extrakt, Typ Nr. 063.007" containing 3.50 weight-% of carnosol, 10.70 weight-% of carnosic acid, 4.30 weight-% of carnosic acid 12-methyl ether, 0.3 weight-% of 20-deoxocarnosol and 0.3 weight-% of rosmanol, based on the total weight of the sage extract (measured by HPLC-UV at 210 nm with the pure substances as reference).

Another sage extract suitable for the purposes of the present invention contains 7.44 weight-% of carnosol, 1.56 weight-% of carnosic acid, 0.23 weight-% of carnosic acid 12-methyl ether, below 0.1 weight-% of 20-deoxocarnosol and 0.41 weight-% of rosmanol, based on the total weight of the sage extract (measured by HPLC-UV at 210 nm with the pure substances as reference).)

Sage extracts suitable for the uses of the present invention may be manufactured according to one of the following two procedures.

### Procedure 1

Dried Salvia officinalis leaves (raw material) are mechanically reduced to small pieces. The raw material is then filled into a pressure stable extraction vessel. Super-critical CO₂ is then running through the extraction vessel at a temperature in the range of from 25 to 75°C (preferably at a temperature of ca. 50°C) and at a high pressure (preferably at a high pressure of 280 bar) in the presence of a small amount of ethanol. Thereby the lipophilic compounds are dissolved. In the stripper the solved compounds (the extract) are separated by a pressure reduction to a pressure in the range of from 40 to 80 bar (preferably by a pressure reduction to 60 bar) at a temperature in the range of from 15 to 45°C (preferably at a temperature of 30°C). The CO₂ may be recycled back into the process. Thus, approximately 1 kg of a sage extract containing carnosol (preferably in an amount of from 2 to 5 weight-%, based on the total weight of the sage extract), camosic acid (preferably in an amount of from 7 to 15 weight-%, based on the total weight of the sage extract), camosic acid 12-methyl ether (preferably in an amount of from 2 to 7 weight-%, based on the total weight of the sage extract), rosmanol (preferably in an amount of from 0.1 to 1 weight-%, based on the total weight of the sage extract) and 20-deoxocamosol (preferably in an amount of from 0.1 to 1 weight-%, based on the total weight of the sage extract) may be obtained of 14 to 20 kg of dried Salvia officinalis leaves.

### Procedure 2

700 g of dried Salvia officinalis leaves are extracted by methanol and then evaporated to dryness. Subsequent solvent-solvent partitionings by use of hexane:H₂O followed by methyl tert-butyl ether (MTBE):H₂O are performed. The MTBE partition is evaporated to dryness. 40 g of this extract may be obtained which yielded enriched amounts of abietanes and triterpenes (camosol, preferably in an amount of from 5 to 10 weight-%; camosic acid, preferably in an amount of from 1 to 3 weight-%; camosic acid 12-methyl ether, preferably in an amount of from 0.05 to 0.5 weight-%; rosmanol, preferably in an amount of from 0.1 to 1 weight-%; and 20-deoxocamosol, preferably in an amount below 0.1 weight-%; all amounts based on the total weight of the sage extract). An optional MPLC chromatography can be applied afterwards to remove oleanolic acid, betulinic acid, or ursolic acid from the extract (yield 25 g).

To the person skilled in the art also other extraction methods are known which yield a sage extract enriched in camosic acid, camosol and camosic acid 12-methyl ether and preferably also in rosmanol and/or 20-deoxocarnosol.

"Camosol" means the racemic mixture as well as pure (*4*α*R,9S,10*α*S*)-carnosol or pure (*4*α*S,9R,10*α*R*)-carnosol or any mixture or diastereoisomer of them. (*4*α*R,9S,10*α*S*)-Camosol is preferred.

"Camosic acid" means the racemic mixture as well as pure (*4*α*R,10*α*S*)-carnosic acid or pure *(4*α*S, 10*α*R*)-carnosic acid or any mixture or diastereoisomer of them. Preferred is (*4*α*R, 10*α*S*)-carnosic acid.

"Camosic acid 12-methyl ether" means the racemic mixture as well as pure (*4*α*R, 10*α*S*)-camosic acid 12-methyl ether or pure (*4*α*S*, *10*α*R*)-carnosic acid 12-methyl ether or any mixture or diastereoisomer of them. Preferred is (*4*α*R*, *10*α*S*)-carnosic acid 12-methyl ether.

"Rosmanol" means the racemic mixture as well as pure *(4*α*R, 9S, 10*α*S*)-rosmanol or pure *(4*α*S, 9R, 10*α*R*)-rosmanol or any mixture or diastereoisomer of them. *(4*α*R, 9S, 10*α*S)-*Rosmanol is preferred.

"20-Deoxo-camosol" means the racemic mixture as well as pure (*4*α*R,9S,10*α*S*)-20-deoxo-carnosol or pure (*4*α*S,9R,10*α*R*)-20-deoxo-carnosol or any mixture or diastereoisomer of them. (*4*α*R,9S,10*α*S*)-20-deoxo-carnosol is preferred.

The dietary compositions according to the present invention may further contain protective hydrocolloids, binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

The term "dietary compositions" comprises any type of (fortified) food/feed and beverages including also clinical nutrition, and also dietary supplements.

Beside a pharmaceutically acceptable carrier and a sage extract containing the mixture of the tricyclic diterpene (derivative)s with the definitions and preferences as described above, the pharmaceutical compositions according to the present invention may further contain conventional pharmaceutical additives and adjuvants, excipients or diluents, flavoring agents, preservatives, stabilizers, emulsifying agents, buffers, lubricants, colorants, wetting agents, fillers, and the like. The carrier material can be organic or inorganic inert carrier material suitable for oral/parenteral/injectable administration.

The dietary and pharmaceutical compositions according to the present invention may be in any galenic form that is suitable for administrating to the animal body including the human body, especially in any form that is conventional for oral administration, e.g. in solid form such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be filled into hard or soft shell capsules. Examples for other application forms are forms for transdermal, parenteral or injectable administration. The dietary and pharmaceutical compositions may be in the form of controlled (delayed) release formulations.

Examples for fortified food are cereal bars, bakery items such as cakes and cookies.

Beverages encompass non-alcoholic and alcoholic drinks as well as liquid preparations to be added to drinking water and liquid food. Non-alcoholic drinks are e.g. soft drinks, sport drinks, fruit juices, lemonades, near-water drinks (i.e. water based drinks with a low calorie content), teas and milk based drinks. Liquid food are e.g. soups and dairy products (e.g. muesli drinks).

The sage extract containing the mixture of the tricyclic diterpene (derivative)s with the definitions and preferences as described above can be used for the manufacture of compositions/medicaments for the treatment of a disorder connected to impaired neurotransmission.

In the context of this invention "treatment" also encompasses co-treatment as well as prevention. "Prevention" can be the prevention of the first occurrence (primary prevention) or the prevention of a reoccurence (secondary prevention).

Thus, the present invention is also directed to a method for the prevention of a disorder connected to impaired neurotransmission in animals including humans, said method comprising administering an effective dose of a sage extract containing the mixture of the tricyclic diterpene (derivative)s with the definitions and preferences as described above, to animals including humans which are in need thereof. In this regard an effective dose of a sage extract containing the mixture of the tricyclic diterpene (derivative)s with the definitions and preferences as described above, may especially be used for maintaining the mental well-being, for maintaining a balanced cognitive function, for helping to reduce the risk of mood swings, for helping to retain a positive mood and for supporting cognitive wellness, and for helping to maintain a good sleep quality.

In the context of this invention the term "disorder" also encompasses diseases.

Medicaments/Compositions for the treatment of disorders connected to impaired neurotransmission encompass antidepressants, mood/vitality improvers, stress relievers, condition improvers, anxiety reducers and obsessive-compulsive behaviour reducers, relaxants, sleep improvers and/or insomnia alleviators. They all improve, enhance and support the physiological neurotransmission, especially in the central nervous system, and therefore alleviate mental malfunction.

Antidepressants are medicaments/compositions for treating mental, behavioural and emotional/affective, neurotic, neurodegenerative, eating and stress related disorders such as e.g. unipolar depression, bipolar depression, acute depression, chronic depression, subchronic depression, dysthymia, postpartum depression, premenstrual dysphoria/syndrom (PMS), climacteric depressive symptoms, aggression, attention deficit disorders (ADS), social anxiety disorders, seasonal affective disorders, anxiety (disorders) such as generalized anxiety disorder (GAD), fibromyalgia syndrome, post-traumatic stress disorders, panic disorders, obsessive compulsive disorders, restless leg syndrome, nervousness, migraine/primary headaches and pain in general, emesis, bulimia, anorexia nervosa, binge eating disorder, gastrointestinal disorders, burn out syndrome, and irritability.

Antidepressants can also be used for (the manufacture of compositions for) primary and secondary prevention and/or the treatment of neurocognitive impairment. Furthermore they are also effective in the treatment of depressive symptoms or other symptoms related to disturbed neurotransmission occurring as comorbidity in chronic diseases such as cardiovascular diseases, strokes, cancer, Alzheimer disease, Parkinson disease, and others.

The sage extracts containing the mixtures of the tricyclic diterpene (derivative)s with the definitions and preferences as described above, and dietary/pharmaceutical compositions containing them are thus suitable for the treatment of animals including humans.

Especially pet animals and farm animals can be in conditions in need of enhanced or improved neurotransmission. Such conditions e.g. occur after capture or transport or with housing, when the animals develop analogous disorders and are distressed or aggressive, or display stereotypic behaviour, or anxiety and obsessive-compulsive behaviour.

Thus, the sage extracts containing the mixtures of the tricyclic diterpene (derivative)s with the definitions and preferences as described above can be used in general as antidepressants for animals including humans, preferably for humans, pet animals and farm animals.

In a further embodiment of the present invention the sage extracts containing the mixtures of the tricyclic diterpene (derivative)s with the definitions and preferences as described above find use as mood improver in general as well as for the manufacture of compositions (such as dietary/pharmaceutical compositions, food, beverages) for such use. "Mood improver" or "emotional wellness booster" or "vitality improver" means that the mood of a person treated with it is enhanced, that the self esteem is increased and/or that negative thoughts and/or negative tension are/is reduced. It also means the emotions are balanced and/or that the general, especially the mental, well being and vitality is improved or maintained, as well as that the risk of mood swings is (helped to be) reduced and that the positive mood is (helped to be) retained.

The sage extracts containing the mixtures of the tricyclic diterpene (derivative)s with the definitions and preferences as described above can also be used in general as anxiety reducer and/or obsessive-compulsive behaviour reducer for animals including humans, preferably for humans, pet animals and farm animals.

Anxiety reducer means that chronic tension and anxious worrying and tension are alleviated and relieved. Hypervigilance syndrome, including restlessness, muscle tension, and sleep problems are relieved. Social and other phobias are resolved. In general, the social environment is experienced less threatening. The person is emotionally relaxed, experiences comfort and enjoys company and contact to other people.

"Relaxant" or "sleep improver" or "insomnia alleviator" means improving sleep onset and helping a person to easily enter sleep, to maintain an undisrupted sleep over the night. It also means to correct circadian rhythm associated sleep disturbances due to jet-lag or shift work, and to prevent and abolish the symptoms associated with sleeplessness, i.e. impairment of cognitive function and memory, mental and physical fatigue, dreaminess, and improve overall quality of life and vital energy.

Moreover, the sage extracts containing the mixtures of the tricyclic diterpene (derivative)s with the definitions and preferences as described above as well as compositions comprising an effective dose of them are useful for the treatment, prevention and the alleviation of stress related symptoms, for the treatment, prevention and alleviation of symptoms related to working overload, exhaustion and/or burn out, for the increase of the resistance or tolerance to stress and/or to favor and facilitate the relaxation in normal healthy individuals i.e. such compositions have an effect as "stress reliever".

Furthermore, sage extracts containing the mixtures of the tricyclic diterpene (derivative)s with the definitions and preferences as described above as well as compositions comprising an effective dose of them are useful for the treatment, prevention and alleviation of anxiety and obsessive-compulsive behaviour in humans and animals.

A further embodiment of the present invention relates to the use of sage extracts containing the mixtures of the tricyclic diterpene (derivative)s with the definitions and preferences as described above and to the use of compositions containing them (such as dietary/pharmaceutical compositions, food, beverages) as "condition improver", i.e. as means to reduce irritability and tiredness, to reduce or prevent or alleviate physical and mental fatigue, and to increase energy in more general terms, especially to increase the brain energy production, in diseased or normal healthy individuals. Moreover for cognition improvement in general, and especially for maintenance or improvement of attention and concentration, of the memory and of the capacity for remembering, of the learning ability, of the language processing, of problem solving and of intellectual functioning; for improvement of the short-term memory; for increasing the mental alertness; for enhancing the mental vigilance; for reducing the mental fatigue; for supporting cognitive wellness, for maintaining balanced cognitive function, for the regulation of hunger and satiety as well as for the regulation of motor activity.

The present invention not only refers to sage extracts containing the mixtures of the tricyclic diterpene (derivative)s with the definitions and preferences as described above and their compositions (such as dietary/pharmaceutical compositions, food, beverages containing them) for use as medicaments, especially for the treatment of disorders connected to impaired neurotransmission, but also for the methods for the treatment of such disorders themselves, as already mentioned above.

In an especially preferred embodiment of such method pet animals or farm animals whose disorders are associated with housing, capture or transport are treated and which may appear in form of anxiety or obsessive-compulsive or stereotypic behavior.

For humans a suitable daily dosage of a sage extract with the definitions and preferences as described above for the purposes of the present invention may be so that the mixture of the tricyclic diterpene (derivative)s is within the range of from 0.001 mg per kg body weight to about 20 mg per kg body weight per day. More preferred is a daily dosage of the mixture of the tricyclic diterpene (derivative)s from about 0.01 to about 10 mg per kg body weight, and especially preferred is a daily dosage of the mixture of the tricyclic diterpene (derivative)s from about 0.05 to 5.0 mg per kg body weight.

In solid dosage unit preparations for humans, the mixture of the tricyclic diterpene (derivative)s with the definitions and preferences as described above, is suitably present in an amount in the range of from about 0.1 mg to about 1000 mg, preferably in the range of from about 1 mg to about 500 mg per dosage unit. The amount of a sage extract containing such a mixture of tricyclic diterpene (derivative)s with the definitions and preferences as described above can be calculated accordingly.

In dietary compositions, especially in food and beverages for humans, the mixture of the tricyclic diterpene (derivative)s with the definitions and preferences as described above, is suitably present in an amount in the range of from about 0.0001 (1 mg/kg) to about 5 weight-% (50 g/kg), preferably from about 0.001 % (10 mg/kg) to about 1 weight-%, (10 g/kg) more preferably from about 0.01 (100 mg/kg) to about 0.5 weight-% (5 g/kg), based upon the total weight of the food or beverage. The amount of a sage extract containing such a mixture of tricyclic diterpene (derivative)s with the definitions and preferences as described above can be calculated accordingly.

In food and drinks in a preferred embodiment of the invention the amount of the mixture of the tricyclic diterpene (derivative)s with the definitions and preferences as described above is in the range of from 10 to 30 mg per serving, i.e. 120 mg per kg food or drink. The amount of a sage extract containing such a mixture of tricyclic diterpene (derivative)s with the definitions and preferences as described above can be calculated accordingly.

For animals excluding humans a suitable daily dosage of a mixture of the tricyclic diterpene (derivative)s with the definitions and preferences as described above, for the purposes of the present invention may be within the range of from 0.001 mg per kg body weight to about 1000 mg per kg body weight per day. More preferred is a daily dosage in the range of from about 0.1 mg to about 500 mg per kg body weight, and especially preferred is a daily dosage in the range of from about 1 mg to 100 mg per kg body weight. The amount of a sage extract containing such a mixture of tricyclic diterpene (derivative)s with the definitions and preferences as described above can be calculated accordingly.

The invention is illustrated further by the following examples.

### Examples

Three different sage extracts were used in the experiments described below.

Sage Extract A was obtained from FLAVEX Naturextrakte GmbH, Rehlingen, Germany ("Salbei Extrakt, Typ Nr. 063.001 ). It contained 0.06 weight-% of carnosol, 0.14 weight-% of camosic acid and 0.36 weight-% of camosic acid 12-methyl ether, based on the total weight of the sage extract and measured by HPLC-UV (High Pressure Liquid Chromatography-Ultraviolet) at 210 nm with the pure substances as reference.

Sage Extract B was obtained from FLAVEX Naturextrakte GmbH, Rehlingen, Germany ("Salbei Antioxidans Extrakt, Typ Nr. 063.007). It contained 3.50 weight-% of carnosol, 10.70 weight-% of camosic acid, 4.30 weight-% of carnosic acid 12-methyl ether, 0.3 weight-% of 20-deoxocarnosol and 0.3 weight-% of rosmanol, based on the total weight of the sage extract and measured by HPLC-UV at 210 nm with the pure substances as reference. The extract was produced as follows: Dried Salvia officinalis leaves (raw material) are mechanically reduced to small pieces. Then the extraction is carried out. Thus, the raw material is filled into a pressure stable extraction vessel. Then super-critical CO₂ is put into the vessel at a temperature of 50°C and at a high pressure of 280 bar extraction in the presence of a small amount of ethanol. Thereby the lipophilic compounds are dissolved. In the stripper the solved compounds (the extract) are separated by a pressure reduction to 60 bar at a temperature of 30°C. The CO₂ is recycled back into the process. Approximately 1 kg of the sage extract B is obtained of 14 to 20 kg of dried Salvia officinalis leaves.

Sage Extract C contained 7.44 weight-% of carnosol, 1.56 weight-% of camosic acid, 0.23 weight-% of camosic acid 12-methyl ether, below 0.1 weight-% of 20-deoxocarnosol and 0.41 weight-% of rosmanol, based on the total weight of the sage extract and measured by HPLC-UV at 210 nm with the pure substances as reference. The sage extract C was manufactured according to the following procedure: 700 g of the dried biomaterial (Salvia officinalis leaves) was extracted by methanol and then evaporated to dryness. Subsequent solvent-solvent partitionings by use of hexane:H₂O followed by methyl tert-butyl ether (MTBE):H₂O were performed. The MTBE partition was evaporated to dryness. 40 g of this extract were obtained which yielded enriched amounts of abietanes and triterpenes.

### Example 1: Serotonin uptake inhibition by sage extracts containing tricyclic diterpene (derivative)s

HEK-293 cells stably expressing the human serotonin re-uptake transporter (hSERT) were obtained from R. Blakely, Vanderbilt University, USA. The cells were routinely grown in Dulbeco's Modified Eagles Medium (Bioconcept) containing 10% fetal calf serum, penicillin, streptomycin, L-glutamine and the antibiotic G418 and passaged by trypsinisation. On the day of assay, cells from 80% confluent flasks were harvested by gentle washing with warm phosphate buffered saline (PBS). Cells were then washed once by centrifugation and re-suspended in Krebs Ringers bicarbonate buffer (Sigma) supplemented with 35 µM pargyline, 2.2 mM CaC1₂, 1 mM ascorbic acid and 5 mM N-2-hydroxyethylpiperazine-N'-2-ethanesulfonic acid (buffer called "Hepes") at a concentration of 10,000 cells in 160 ul of buffer, and aliquoted into round bottomed polypropylene 96 well microtitre plates (Coming) at 10,000 cells per well. Serotonin uptake into the cells was determined by addition of radio-labeled (3H) serotonin (GE Healthcare) to a concentration of 20 nM, and incubation for 40 minutes at 37°C with gentle shaking. At the end of this time unincorporated label was removed by filtration though Unifilter 96 GF/B plates (Perkin Elmer) using a Tomtec Mach III M cell harvester. The incorporated serotonin retained on the plates was quantified by liquid scintillation counting using Microscint-40 / Topcount (Perkin Elmer).

The effect of the sage extracts A, B and C on the serotonin uptake was determined by their inclusion in the assay at a range of concentrations between 0.03 and 100 µM for 10 minutes prior to and during the addition of (3H) serotonin. Serotonin uptake via the transporter was inhibited by the sage extracts A, B and C in a concentration dependent manner. The calculated IC₅₀ values for inhibition of serotonin uptake by the sage extracts A, B and C are shown in Table 1.

**Table 1: Inhibition of serotonin uptake into transfected HEK-293 cells by sage extracts A, B and C**

| **Substance** | **IC₅₀** for Tritiated Serotonin Uptake [µM unless otherwise stated] |
|---|---|
| Sage Extract A | 19.0 ug/ml +/- 2.8 s.e.m. n = 2 |
| Sage Extract B | 11.3 ug/ml +/- 2.3 s.e.m. n = 3 |
| Sage Extract C | 23.5 ug/ml +/- 7.3 s.e.m. n = 2 |

The mean value of the n independent IC₅₀ determinations +/- the standard error in the mean (s.e.m.) is shown.

### Example 2: Monoaminooxidase inhibition by sage extracts containing tricyclic diterpene (derivative)s

The organic amines p-tyramine or benzylamine were used as substrates for the Monoamine oxidase A (MAO-A) and B (MAO-B) enzymes respectively. The H₂O₂ produced by this reaction was quantified by reaction with vanillic acid, catalysed by horse radish peroxidase (HRP).

The reactions were carried out in polystyrene microtitre plates. The MAO enzymes (final concentration 2 U/ml) were mixed with either p-tyramine (Sigma, final concentration 0.5 mM) or benzylamine (Sigma, final concentration 0.5 mM) as appropriate and the chromogenic solution (containing vanillic acid (Fluka), 4-aminoantipyrine (Fluka) and horse radish peroxidase (Sigma), final concentrations 0.25 mM, 0.125 mM and 1 U/ml respectively) in 0.2 M potassium phosphate buffer pH 7.6. The reactions were followed in a microtitre plate absorbance reader eg Spectramax M5 (Molceular Devices Corporation). Absorbance readings at 495 nm were taken every 15 seconds for 40 minutes and the initial reaction velocities calculated by linear regression using SOFTmaxPro (Molecular Devices Corporation).

The effect of the sage extracts A, B and C on the monoamine oxidase enzymes was determined by their inclusion in the assay at a range of concentrations between 0.03 and 100 µM for 10 minutes prior to and during the incubation with substrate. To determine the effect of the extracts on the HRP catalyzed portion of the reaction, the MAO enzyme was replaced by H₂O₂ (Molecular Probes, final concentration 0.2 mM). The reactions containing MAO-A and MAO-B were both inhibited by the sage extracts A, B and C in a concentration dependent manner, whilst the control reaction, was unaffected. The measured IC₅₀ values for inhibition of monoamine oxidase activity by the sage extracts A, B and C are shown in Table 2.

**Table 2: Inhibition of MAO-A and MAO-B by the sage extracts A, B and C**

| **Substance** | **IC50 for Inhibition of MAO-A [µM unless otherwise stated]** | **IC50 for Inhibition of MAO-B [µM unless otherwise stated]** |
|---|---|---|
| Sage Extract A | >> 100 ug/ml n=2 | >> 100 ug/ml n=2 |
| Sage Extract B | 3.66 ug/ml +/- 0.36 s.e.m. n=2 | 7.9 ug/ml +/- 0.9 s.e.m. n=2 |
| Sage Extract C | 5.7 ug/ml +/- 0.3 s.e.m. n=3 | 18.9 ug/ml +/-1.5 s.e.m. n=2 |

The mean value of the n independent IC₅₀ determinations +/- the standard error in the mean (s.e.m.) is shown.

### Example 3: Marble burying test

"Defensive burying" behaviour was demonstrated by rats burying noxious objects, such as drinking spouts filled with a unpleasant-tasting liquid (WILKIE, D.M., MACLENNAN, A.J. & PINEL, J.P.J. (1979). Rat defensive behavior: burying noxious food. Journal of the Experimental Analysis of Behavior, 31, 299-306.) or shock prods (PINEL, J.P.J. & TREIT, D. (1978). Burying as a defensive response in rats. Journal of Comparative and Physiological Psychology, 92, 708-712.). The marble burying test was devised as a modification of such a test. Poling et al. (POLING, A., CLEARY, J. & MONAGHAN, M. (1981). Burying by rats in response to aversive and nonaversive stimuli. Journal of the Experimental Analysis of Behavior, 35, 31-44.) exposed rats to individual cages each containing 25 marbles, daily for 10 or 21 consecutive days. The number of marbles buried, on each day of the 10 day (d) period, or 24 hours (h) after the 21 d exposure, were counted. The authors reported that the burying of marbles was not determined by novelty, or due to any noxious stimuli.

Marble burying behaviour by mice is reported to be sensitive to a range of minor (e.g. diazepam) and major (e.g. haloperidol) tranquilisers (BROEKKAMP, C.L., RIJK, H.W., JOLY-GELOUIN, D. & LLOYD, K.L. (1986). Major tranquillizers can be distinguished from minor tranquillizers on the basis of effects on marble burying and swim-induced grooming in mice. European Journal of Pharmacology, 126, 223-229.), in addition to SSRIs (e.g. fluvoxamine, fluoxetine, citalopram), tricyclic antidepressants (e.g. imipramine, desipramine) and selective noradrenaline uptake inhibitors (e.g. reboxetine), at doses which do not induce sedation. The model may reflect either anxiety-like- or obsessive-compulsive- behaviour (see DE BOER, S.F. & KOOLHAAS, J.M. (2003). Defensive burying in rodents: ethology, neurobiology and psychopharmacology. European Journal of Pharmacology, 463, 145-161.).

The method applied here follows that described by Broekkamp et al. (European Journal of Pharmacology 1986, 126, 223-229.). Mice (n = 15 per treatment group) were individually placed in transparent plastic cages (33 x 21 x 18 cm) with 5 cm of sawdust on the floor and 25 marbles (diameter 1 cm) grouped in the centre of the cage. A second, up-turned, cage served as a lid. The number of marbles covered by sawdust (by at least two-thirds) was counted at the end of the 30-minute test period. Tests were performed by investigators blind to the drug treatment protocol.

Prior to testing, all test cages and marbles were "impregnated" by leaving 10 naive mice in each cage for 15 minutes.

Sage extract B was investigated after oral application (24, 5 and 1 h before the test) in the Marble Burying test in the mouse to evaluate its potential anxiolytic effects.

Sage extract was administered at doses of 30, 100 and 300 mg/kg, and showed a significant effect in the Marble Burying test. At the highest dose of 300mg/kg we observed a 41 % decreased number of marbles covered compared with vehicle control.

Data were analysed by comparing treated groups with vehicle control using unpaired Student's t-tests.

### Results

**Table 3: Effects of sage extract B, venlafaxine, fluoxetine and clobazam in the marble burying test in the mouse (15 mice per group)**

| TREATMENT (mg/kg) p.o. -24 h and -5 h | TREATMENT (mg/kg) p.o. -1 h | NUMBER OF MARBLES COVERED BY SAWDUST | | |
|---|---|---|---|---|
| | | mean ± s.e.m. | p value | % change from control |
| Vehicle | Vehicle | 17.9 ± 2.1 | | |
| sage extract B (30) | sage extract B (30) | 14.0± 2.3 NS | 0.2266 | -22% |
| sage extract B (100) | sage extract B (100) | 14.9 ± 2.2 NS | 0.3419 | -17% |
| sage extract B (300) | sage extract B (300) | 10.6 ± 2.6 * | 0.0374 | -41% |
| Venlafaxine (24) | Venlafaxine (24) | 9.5 ± 1.8 ** | 0.0058 | -47% |
| Fluoxetine (10) | Fluoxetine (10) | 7.6 ± 1.6 *** | 0.0006 | -58% |
| Vehicle | Clobazam (32) | 2.9 ± 1.2 *** | <0.0001 | -84% |

| | | | | |
|---|---|---|---|---|
| Student's t test: NS = Not Significant; * = p < 0.05; ** = p < 0.01; *** = p < 0.001 #: dead after the first administration (1/15) and after the second administration (1/15). | | | | |

The highest dose of sage extract B tested (300 mg/kg) clearly and significantly reduced marble burying behaviour, in a similar manner to the serotonin and norepinephrine re-uptake inhibitor venlafaxine.

### Example 4: Effect of sage extract B in the Light-Dark Box Test in the mouse after subchronic, oral gavage

The method, which detects anxiolytic activity, follows that described by Crawley (Pharmacol. Biochem. Behav., 15, 695-699, 1981*).* Anxiolytics increase the time spent in the light compartment.

Animals were placed into the light compartment of a 2-compartment box with one half light and open (25 x 27 x 27 cm) and the other half dark and closed (20 x 27 x 27 cm). The time spent in each compartment as well as the number of times the animal crosses from one side to the other is scored during a 3-minute test. 15 mice were studied per group. The test was performed blind.

Sage extract B was evaluated at 3 doses (30, 100, 300 mg/kg BW), administered by oral gavage (per os, p.o.) 24 hours, 5 hours and 1 hour before the test, and compared with a vehicle control group (corn oil). The sage extract B was dissolved in corn oil ("vehicle"). Clobazam (32 mg/kg p.o.), dispersed in 0.2% w/v hydroxypropylmethyl cellulose in distilled water), administered 1 hour before the test, was used as reference substance. Mice in this group received additional administrations of vehicle at 24 hours and 5 hours before the test in order to maintain experimental blinding.

### Results

**Table 4: Effects of sage extract B and clobazam in the light-dark box test in the mouse (15 mice per group)**

| Sage extract B (mg/kg) p.o. -24 h and -5 h | Sage extract B (mg/kg) p.o. -1 h | NUMBER OF CROSSINGS | | | TIME SPENT IN LIGHT COMPARTMENT (s) | | |
|---|---|---|---|---|---|---|---|
| | | mean ± s.e.m. | p value | % change from control | mean ± s.e.m. | p value | % change from control |
| Vehicle | Vehicle | 11.2 ± 1.3 | | | 71.7 ± 4.9 | | |
| 30 | 30 | 12.8 ± 1.6 NS | 0.4407 | +14% | 73.7 ± 5.3 NS | 0.7909 | +3% |
| 100 | 100 | 9.7 ± 1.0 NS | 0.3739 | -13% | 70.3 ± 6.5 NS | 0.8582 | -2% |
| 300 | 300 #1 #2 | 12.6 ± 1.7 NS | 0.5133 | +13% | 101.1 ± 11.0 * | 0.0187 | +41% |
| Vehicle | Clobazam 32 mg/kg p.o. -1 h | 11.9 ± 1.8 NS | 0.7405 | +6% | ** 125.5 ± 6.4 * | <0.0001 | +75% |

| | | | | | | | |
|---|---|---|---|---|---|---|---|
| Student's t test: NS = Not Significant; * = p < 0.05; *** = p < 0.001 #1: dead after p.o. -1 h administration (false administration) (1/15). #2: sedation sage extract B at 300 mg/kg (1/14) and clobazam (2/15). | | | | | | | |

The highest dose of sage extract B tested (300 mg/kg) clearly and significantly increased the time spent in the light compartment compared to vehicle control. Thus, the sage extract B has an anxiolytic activity at 300 mg/kg.

### Example 5: Preparation of a soft gelatin capsule

A soft gelatin capsule (500 mg) is prepared comprising the following ingredients:

| **Ingredient** | **Amount per Capsule** |
|---|---|
| A mixture containing carnosic acid, carnosol and camosic acid 12-methyl ether | 200 mg |
| Lecithin | 50 mg |
| Soy bean oil | 250 mg |

Two capsules per day for 3 months may be administered to a human adult for the treatment of mild chronic dysthymia.

### Example 6: Preparation of a soft gelatin capsule

A soft gelatin capsule (600 mg) is prepared comprising the following ingredients:

| **Ingredient** | **Amount per Capsule** |
|---|---|
| A mixture containing camosic acid, carnosol and camosic acid 12-methyl ether | 200 mg |
| Evening prim rose oil | 300 mg |
| Vitamin B₆ | 100 mg |

One capsule per day preferably at the second half of the menstrual cycle should be taken for 14 days for the treatment of premenstrual syndrome and premenstrual dysphoric disorder.

### Example 7: Preparation of a tablet

A 400 mg-tablet is prepared comprising the following ingredients:

| | |
|---|---|
| **Ingredient** | **Amount per tablet** |
| A mixture containing camosic acid, carnosol and camosic acid 12-methyl ether | 100 mg |
| Passion flower standardized extract | 150 mg |
| Green Tea Extract, e.g. TEAVIGO® from DSM Nutritional Products, Kaiseraugst, Switzerland | 150 mg |

For general well being, energizing and stress alleviation, one tablet is taken twice daily for 3 months.

### Example 8: Preparation of an instant flavoured soft drink

| **Ingredient** | **Amount [g]** |
|---|---|
| A mixture containing camosic acid, carnosol and carnosic acid 12-methyl ether | 0.9 |
| Sucrose, fine powder | 922.7 |
| Ascorbic acid, fine powder | 2.0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4 |
| **Total amount** | **1000** |

All ingredients are blended and sieved through a 500 µm sieve. The resulting powder is put in an appropriate container and mixed on a turbular blender for at least 20 minutes. For preparing the drink, 125 g of the obtained mixed powder are taken and filled up with water to one liter of beverage.

The ready-to-drink soft drink contains ca. 30 mg of the tricyclic diterpene (derivative) mixture per serving (250 ml).
As a strenghtener and for general well being 2 servings per day (240ml) should be drunk.

### Example 9: Preparation of a fortified non baked cereal bar

| **Ingredient** | **Amount [g]** |
|---|---|
| A mixture containing carnosic acid, carnosol and camosic acid 12-methyl ether | 0.95 |
| Sugar | 114.55 |
| Water | 54.0 |
| Salt | 1.5 |
| Glucose syrup | 130.0 |
| Invert sugar syrup | 95.0 |
| Sorbitol Syrup | 35.0 |
| Palmkernel fat | 60.0 |
| Baking fat | 40.0 |
| Lecithin | 1.5 |
| Hardenend palm-oil | 2.5 |
| Dried and cut apple | 63.0 |
| Cornflakes | 100.0 |
| Rice crispies | 120.0 |
| Wheat crispies | 90.0 |
| Roasted hazelnut | 40.0 |
| Skim milk powder | 45.0 |
| Apple flavour 74863-33 | 2.0 |
| Citric acid | 5.0 |
| **Total amount** | **1000** |

The tricyclic diterpene (derivative) mixture is premixed with skim milk powder and placed in a planetary bowl mixer. Cornflakes and rice crispies are added and the total is mixed gently. Then the dried and cut apples are added. In a first cooking pot sugar, water and salt are mixed in the amounts given above (solution 1). In a second cooking pot glucose, invert and sorbitol syrup are mixed in the amounts given above (solution 2). A mixture of baking fat, palmkernel fat, lecithin and emulsifier is the fat phase. Solution 1 is heated to 110°C. Solution 2 is heated to 113°C and then cooled in a cold water bath. Afterwards solution 1 and 2 are combined. The fat phase is melted at 75°C in a water bath. The fat phase is added to the combined mixture of solution 1 and 2. Apple flavour and citric acid are added to the liquid sugar-fat mix. The liquid mass is added to the dry ingredients and mixed well in the planetary bowl mixer. The mass is put on a marble plate and rolled to the desired thickness. The mass is cooled down to room temperature and cut into pieces. The non baked cereal bar contains ca. 25 mg of the tricyclic diterpene (derivative) mixture per serving (30 g). For general wellbeing and energizing 1-2 cereal bars should be eaten per day.

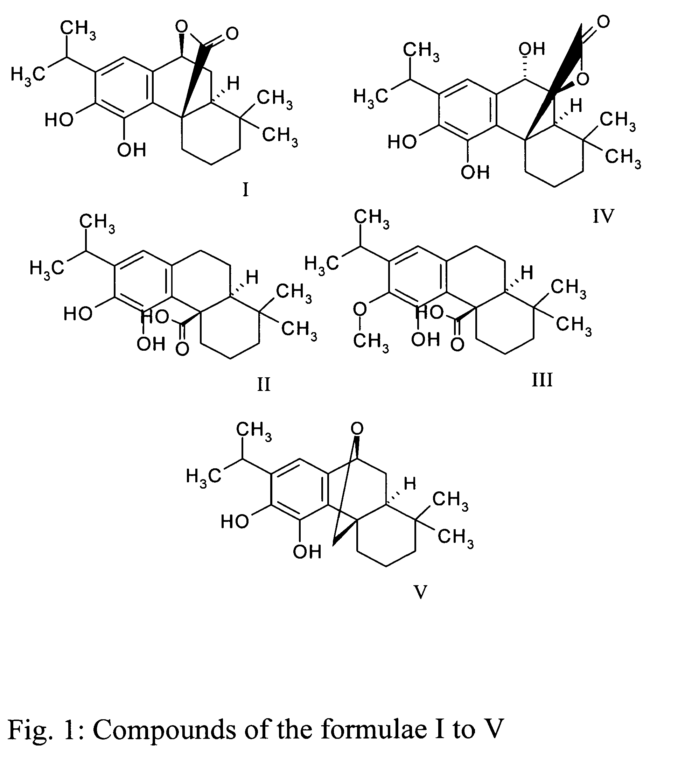

## Claims

1. A dietary or pharmaceutical composition comprising a sage extract comprising carnosol, camosic acid and carnosic acid 12-methyl ether.

2. The dietary or pharmaceutical composition according to claim 1 further comprising rosmanol and/or 20-deoxocarnosol.

3. The dietary or pharmaceutical composition according to claim 2, wherein the sage extract further comprises rosmanol and/or 20-deoxocarnosol.

4. The dietary composition according to any one of claims 1 to 3 being in form of food such as dairy products (yoghurts), in form of fortified food such as cereal bars and bakery items such as cakes and cookies, in form of dietary supplements such as tablets, pills, granules, dragées, capsules, and effervescent formulations, in form of non-alcoholic drinks such as soft drinks, sport drinks, fruit juices, lemonades, near-water drinks, teas and milk based drinks, in form of liquid food such as soups and dairy products (muesli drinks).

5. Use of the dietary composition according to any one of claims 1 to 4 or the pharmaceutical composition according to any one of claims 1 to 3 as antidepressant, mood/vitality improver, stress reliever, condition improver, reducer of anxiety, reducer of obsessive-compulsive behaviour, relaxant, sleep improver and/or insomnia alleviator.

6. Sage extract as defined in claim 1 or 3 for use as medicament.

7. The sage extract as defined in claim 1 or 3 for use as medicament for the treatment of a disorder connected to impaired neurotransmission.

8. The sage extract as defined in claim 1 or 3 for use according to claim 7 as antidepressant, mood/vitality improver, stress reliever, condition improver, reducer of anxiety, reducer of obsessive-compulsive behaviour, relaxant, sleep improver and/or insomnia alleviator.

9. Use of a sage extract as defined in claim 1 or 3 for the manufacture of a composition for the treatment of a disorder connected to impaired neurotransmission.

10. The use according to claim 9, wherein the composition is an antidepressant, a mood/vitality improver, a stress reliever, a condition improver, a reducer of anxiety, a reducer of obsessive-compulsive behaviour, a relaxant, a sleep improver and/or an insomnia alleviator.

11. A method for the treatment of a disorder connected to impaired neurotransmission in animals including humans, said method comprising administering an effective dose of a sage extract as defined in claim 1 or 3 to animals including humans which are in need thereof.

12. The method according to claim 11, wherein the animal is a human, a pet animal or a farm animal.
